# EUROPEAN PATENT APPLICATION

(11) **EP 1 363 345 A2**
(43) Date of publication of application: **19.11.2003**
(21) Application number: 03252897.8
(22) Date of filing: 09.05.2003
(51) Int. Cl.: H01M 10/39, H01G 9/02, C08K 5/31, C07C 279/02

(54) **Guanidine derivatives as cations for ambient temperature molten salts in electrochemical power sources**

(30) Priority: 09.05.2002 US 379096 P
(71) Applicant: Wilson Greatbatch Technologies, Inc., Clarence, New York 14031 (US)
(72) Inventor: Schlaikjer, Carl R., Concord, Massachusetts 01742 (US)
(74) Representative: Colmer, Stephen Gary

(57) **Abstract**

The present invention is directed to an ambient temperature molten salt as non-aqueous electrolyte. The molten salt comprises a cation of a guanidine moiety and an anion. The cation is selected from alkyl groups, alicyclic groups, or aromatic groups attached asymmetrically to guanidine. An exemplary salt is tetramethylguanidinimum bis-trifluoromethanesulfonyl imide, which is liquid at ambient temperature and only slightly soluble in water. The salt is prepared by bringing together two aqueous salt solutions, one containing tetramethylguanidine hydrochloride, and the other containing lithium *bis*-trifluoromethanesulfonyl imide. The electrolyte is useful with electrochemical devices such as primary and secondary electrochemical cells and capacitors, such as of the electrolytic and electrolytic/electrochemical hybrid types.

## Description

### CROSS-REFERENCE TO RELATED APPLICATION

This application claims priority from provisional application Serial No. 60/379,096, filed May 9, 2002.

### BACKGROUND OF THE INVENTION

### 1. Field of the Invention

This invention relates to electrochemical power sources such as cells, batteries and capacitors. More particularly, the present invention is directed to ambient temperature molten salts that are useful as electrolytes in primary and secondary electrochemical cells and in high energy density electrolytic capacitors. Additionally, such salts are useful as hydraulic fluids and fire retardants.

### 2. Prior Art

Examples of electrolytes currently being used in rechargeable electrochemical power sources include liquid, gel, and dry polymer types. Dry polymer electrolyte cells without plasticizers exist, but their inadequate conductivity and low lithium ion transference prevent them from being used at ambient or reduced temperatures.

Liquid and gel electrolytes have higher ionic conductivity and adequate lithium ion transference when compared with dry polymer electrolytes. An example is a solvent system of propylene carbonate and 1,2-dimethoxyethane having a lithium salt such as LiPF₆ or LiAsF₆ dissolved therein. Such as electrolyte is typically used to activate a lithium/silver vanadium oxide (Li/SVO) cell. Additionally, liquid and gel electrolyte cells, such as of a carbonaceous negative electrode and a lithium cobalt oxide positive electrode, are capable of cycling at relatively high rates and low temperatures. One major disadvantage with them, however, is that organic solvents must be included in the electrolyte to improve conductivity and, in the case of the liquid phase, lower viscosity. Liquid and gel electrolytes are also relatively volatile and flammable, which poses a risk of fire when they are heated. In addition, liquid and gel electrolyte cells, whether of a primary or a secondary chemistry, are subject to gassing and subsequent leakage. The packaging and processing required to prevent leakage is complex and, therefore, costly.

In contrast, electrolytes based on ambient temperature molten salts promise the safety of dry polymers along with substantially higher ionic conductivies. One example is described in U.S. Patent No. 5,827,602 to Koch et al., which relates to derivatives of imidazole and the usefulness of these ambient temperature molten salts as electrolytes for high energy density batteries and capacitors. Pyridine and other five and six membered heterocyclic cations containing one or more nitrogen atoms are also discussed. For example, 1-methyl-3-ethyl-1 *H*-imidazolium, which is shown below, is a molten salt at ambient temperatures.

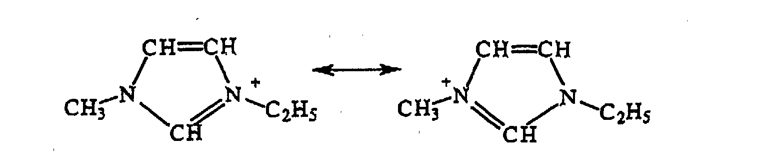

### 1-methyl-3-ethyl-1 H-imidazolium cation

Delocalization of the positive charge on this five-membered heterocyclic ring is made possible by the two illustrated resonant hybrid cation structures. The resonant structures are believed to be one of the properties of such cations responsible for lowering the melting point of their derivative salts. The other reason such salts are molten at ambient temperatures is the asymmetry afforded by the difference in the aliphatic groups attached to the nitrogen atoms.

While derivative salts, such as those taught by Koch et al., are potentially useful as battery electrolytes, they are not without limitations. These include a narrow potential window, their propensity to become intercalated into graphite negative electrodes, and their need to be "blended" with organic solvents, such as carbonate esters, to improve conductivity and lower viscosity.

### SUMMARY OF THE INVENTION

The present invention is directed to the use of a new ambient temperature molten salt as an electrolyte for electrochemical energy storage devices, such as electrochemical cells and electrolytic capacitors. The ambient temperature molten salt comprises a guanidine cation, particularly an asymmetrically substituted one, combined with an anion. In order to increase resistance to electrochemical oxidation and reduction, the substituent organic group is preferably fully protonated, or partially or totally halogenated, such as by fluorine. A particularly preferred anion is lithium bis-trifluoromethanesulfonyl imide.

The product molten salt is used in its liquid form, or is combined with a polymer to provide a gel electrolyte. Either type of nonaqueous electrolyte provides high conductivity in an electrochemical system without the use of volatile components. There is also no risk of fire if the cell or capacitor is overheated or overcharged, even in the absence of safety circuits. This improved safety is without loss in capacity, cycle life, or rate capability relative to the existing technology, such as the above-discussed Koch et al. electrolytes. Cells and capacitors of the present invention are also easier to manufacture and to package than cells and capacitors activated with conventional electrolytes.

These and other objects of the present invention will become increasingly more apparent to those skilled in the art by reference to the following description.

### DETAILED DESCRIPTION OF THE PREFERRED EMBODIMENTS

Guanidine is a unique amine in which three nitrogen atoms are joined to the same carbon atom. As shown in the equation below, because protonation of the unsaturated nitrogen makes possible the delocalization of the positive charge over all three nitrogen atoms, guadinine is one of the strongest organic bases known.

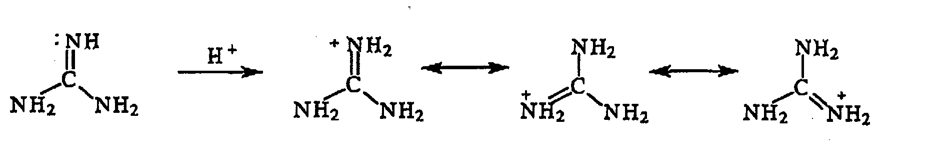

An asymmetric guanidinium cation, namely the protonated form of N,N,N',N'-tetramethyl guanidine, forms an ambient temperature molten salt with an anion, for example *bis*-trifluoromethanesulfonyl imide. The latter compound is itself an ion capable of assuming five resonant hybrid structures, as indicated below.

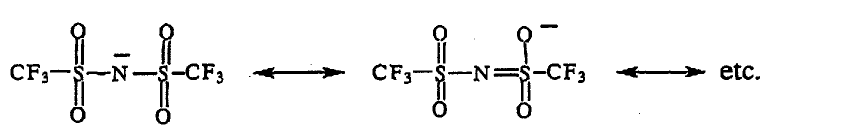

### bis-trifluoromethanesulfonyl imide

The product tetramethylguanidinium *bis*-trifluoromethanesulfonyl imide is liquid at ambient temperature and only slightly soluble in water. Being liquid at ambient temperature means that the electrolyte is in a liquid phase at a temperature of about 60°C, or less.

A lithium salt is preferred for electrochemical cells having lithium as the anode active material. Therefore, one convenient method of preparing this product compound is by reacting two aqueous salt solutions, one containing tetramethylguanidine hydrochloride, the other containing lithium *bis*-trifluoromethanesulfonyl imide.

In a broader sense, however, the present invention is directed to replacing one or more of the protons on the guanidine moiety with a different organic group while maintaining asymmetry. Preferred organic substituents are aliphatic, such as ethyl, propyl, isopropyl, butyl, isobutyl, *sec*-butyl, *tert*-butyl, etc., or halogenated alkyl groups, such as fully or partially halogenated ethyl, propyl, isopropyl, butyl, isobutyl, *sec*-butyl, and *tert*-butyl groups. Halogens include fluorine, chlorine, bromine, iodine and astatine.

An example of an asymmetrically alkyl substituted guanidine is N,N,N',N'-tetramethyl-N",N"-diethylguanidinium.

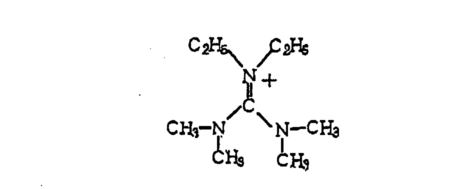

### N, N, N', N'-tetramethyl-N", N"-diethylguanidinium

Alicyclic and aromatic groups are also preferred substituents, especially those including the guanidine nucleus in one, two, or three heterocyclic rings. An example of this type of compound is 1-methyl-7-*n*-propyl-1,5,7-triazabicyclo[4.4.0]dec-5-enium.

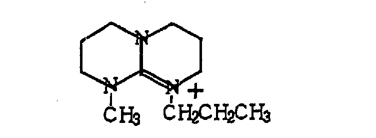

### 1-methyl-7-n-propyl-1, 5, 7-triazabicyclo[4.4.0]dec-5-enium

Besides *bis*-trifluoromethanesulfonyl imide, anions useful in conjunction with a guanidinium salt include PF₆⁻, BF₄⁻, and triflate (CF₃SO₃⁻). Preferred are lithium salts. Other lithium salts useful with the guanidinium cation include lithium salts of: AsF₆⁻, SbF₆⁻, ClO₄⁻, O₂⁻, AlCl₄⁻, GaCl₄⁻, C(SO₂CF₃)₃⁻, N(SO₂CF₃)₂⁻, SCN⁻, O₃SCF₃⁻, C₆F₅SO₃⁻, O₂CCF₃⁻, SO₆F⁻, B(C₆H₅)₄⁻, and mixtures thereof.

Also useful as anions are asymmetric derivatives of bis-trifluoromethanesulfonyl imide, such as trifluoromethanesulsonyltrifluoroacetyl imide and trifluoromethanesulfonylpentafluoroethanesulfonyl imide.

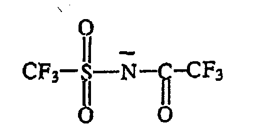

### Trifluoromethanesulsonyltrifluoroacetyl imide

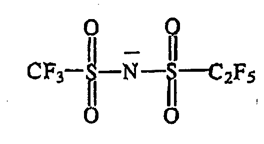

### Trifluoromethanesulfonylpentafluoroethanesulfonyl imide

Other useful anions include any having extensive delocalization of the negative charge, such as the *closo*carborates: B₉H₉CH⁻, B₁₁H₁₁CH⁻, *closo*borates: B₁₀H₁₀²⁻ and B₁₂H₁₂²⁻, and their halogenated derivatives.

If a single-phase gel electrolyte is preferred, the product guanidine derivative molten salt is mixed with a unsaturated monomer. Suitable polymerizerable monomers have at least one α-unsaturated functionality, and more preferably multiple α-unsaturated functionalities, such as multi-functional (meth)acrylates so that they are relatively rapidly curablable inside a cell casing to form a cross-linked matrix or network. Preferably, the (methyl)acryloyl monomer has at least one functional group selected from the group consisting of alkyl, alkyl ether, alkoxylated alkyl and alkoxylated phenol functional groups. Suitable monomers include dipentaerythritol hexaacrylate (DPHA), dipentaerythritol pentaacrylate (DPAA), pentaerythritol tetraacrylate, ethoxylated pentaerythritol tetraacrylate, di(trimethylolpropane) tetraacrylate (DTMPTA), trimethylolpropane trimethacrylate, ethoxylated trimethylolpropane triacrylate (ETMPTA), ethoxylated bisphenol diacrylate, hexanediol diacrylate, and mixtures thereof. For more detail regarding gel electrolytes, reference is drawn to U.S. application Serial No. 10/000,883, filed November 15, 2001. This application is assigned to the assignee of the present invention and incorporated herein by reference.

The present ambient temperature molten salts are useful as electrolytes in a wide variety of electrochemical power sources. These include primary electrochemical cells, such as of the lithium/silver vanadium oxide couple (Li/SVO), Li/copper silver vanadium oxide (Li/CSVO), and lithium/manganese oxide (Li/MnO₂). Exemplary Li/SVO cells are described in U.S. Patent Nos. 4,310,609 and 4,391,729, both to Liang et al., and 5,580,859 to Takeuchi et al. while an exemplary Li/CSVO cell is described in U.S. Patent Nos. 5,472,810 and 5,516,340, both to Takeuchi et al. All of these patents are assigned to the assignee of the present invention and incorporated herein by reference.

The ambient temperature molten salts of the present invention are also useful for activating secondary electrochemical cells. In a secondary system, the negative electrode comprises a material capable of intercalating and de-intercalating the active material, such as the preferred alkali metal lithium. A carbonaceous negative electrode comprising any of the various forms of carbon (e.g., coke, graphite, acetylene black, carbon black, glass carbon, "hairy carbon" etc.) that are capable of reversibly retaining the lithium species is preferred for the negative electrode material. A "hairy carbon" material is particularly preferred due to its relatively high lithium-retention capacity. "Hairy carbon" is a material described in U.S. Patent No. 5,443,928 to Takeuchi et al., which is assigned to the assignee of the present invention and incorporated herein by reference. Graphite is another preferred material. Regardless of the form of the carbon, fibers of the carbonaceous material are particularly advantageous because they have excellent mechanical properties that permit them to be fabricated into rigid electrodes that are capable of withstanding degradation during repeated charge/discharge cycling. Moreover, the high surface area of carbon fibers allows for rapid charge/discharge rates.

Also in secondary systems, the positive electrode preferably comprises a lithiated material that is stable in air and readily handled. Examples of such air-stable lithiated cathode active materials include oxides, sulfides, selenides, and tellurides of such metals as vanadium, titanium, chromium, copper, molybdenum, niobium, iron, nickel, cobalt and manganese. The more preferred oxides include LiNiO₂, LiMn₂O₄, LiCoO₂, LiCo_{0.92}Sn_{0.08}O₂ and LiCo₁₋ₓNiₓO₂.

The present ambient temperature molten salts are not only useful as electrolytes in primary and secondary electrochemical cells, they are useful in capacitors as well. This includes conventional electrolytic capacitors, as well as those of an electrolytic/electrochemical hybrid type. Capacitor cathodes commonly used in electrolytic capacitors include etched aluminum foil in aluminum electrolytic capacitors, and those commonly used in wet tantalum capacitors such as of silver, sintered valve metal powders, platinum black, and carbon. The cathode of hybrid capacitors include a pseudocapacitive coating of a transition metal oxide, nitride, carbide or carbon nitride, the transition metal being selected from the group consisting of ruthenium, cobalt, manganese, molybdenum, tungsten, tantalum, iron, niobium, iridium, titanium, zirconium, hafnium, rhodium, vanadium, osmium, palladium, platinum, and nickel. The pseudocapacitive coating is deposited on a conductive substrate such as of titanium or tantalum. The electrolytic/electrochemical hybrid capacitor has high energy density and is particularly useful for implantable medical devices such as a cardiac defibrillator.

The anode is of a valve metal consisting of the group vanadium, niobium, tantalum, aluminum, titanium, zirconium and hafnium. The anode can be a foil, etched foil, sintered powder, or any other form of porous substrate of these metals.

A preferred chemistry for a hybrid capacitor comprises a cathode electrode of a porous ruthenium oxide film provided on a titanium substrate coupled with an anode of a sintered tantalum powder pressed into a pellet. A suitable separator material impregnated with the present working electrolyte segregates the cathode and anode electrodes from each other. Such a capacitor is described in U.S. Patent Nos. 5,894,403 to Shah et al., 5,920,455 to Shah et al. and 5,926,362 to Muffoletto et al. These patents are assigned to the assignee of the present invention and incorporated herein by reference.

The following example describes the preparation of an ambient temperature salt according to the present invention, and it sets forth the best mode contemplated by the inventors of carrying out the invention, but it is not to be construed as limiting.

### EXAMPLE I

Tetramethylguanidinium *bis*-trifluoromethanesulfonyl imide was prepared as follows. 7.1 mL of tetramethyl guanidine (6.54 grams; 57 mmoles) were dissolved in about 25 mL of water. To this solution were slowly added with stirring 5 mL (60 mmoles) of concentrated (12 molar) hydrochloric acid. In a separate vessel, 16.3 grams (57 mmoles) of lithium *bis*-trifluoromethanesulfonyl imide were dissolved in about 25 mL of water. Both solutions were added to a 125 mL separatory funnel and agitated. The mixture was allowed to separate, and the denser molten salt was drawn off the bottom. The molten salt was washed twice in the separatory funnel with two 25 mL portions of water. This produced about 13 mL (19 grams) of tetramethylguanidinium *bis*-trifluoromethanesulfonyl imide as a crystal clear, colorless liquid. The yield was about 85%.

By "asymmetrically substituted" is meant herein that the resulting guanidinium ion does not have all three nitrogen atoms identically substituted.

## Claims

1. A salt which is molten at ambient temperature and which comprises;
(a) a guanidine cation; and
(b) an anion.

2. A salt according to claim 1 wherein the guanidine cation includes at least one organic substituent.

3. A salt according to claim 1 or 2 wherein the guanidine cation is an asymmetrically substituted guanidinium ion.

4. A salt according to claim 2 or claim 3 wherein the, or at least one of the, substituents is a substituted or unsubstituted, aliphatic, alicyclic or aromatic group.

5. A salt according to claim 4 wherein the, or at least one of the, substituents is a substituted or unsubstituted C₁ to C₆ alkyl group.

6. A salt according to claim 5 wherein the, or at least one of the, substitutents is selected from the group consisting of ethyl, propyl, isopropyl, butyl, isobutyl, sec-butyl, and tert-butyl, or is selected from the group consisting of at least partially halogenated ethyl, propyl, isopropyl, butyl, isobutyl, *sec*-butyl, and tert-butyl.

7. A salt of claim 6 wherein the halogen is selected from the group consisting of fluorine, chlorine, bromine, iodine and mixtures thereof.

8. A salt according to claim 4 wherein the, or at least one of the, substituents is a multivalent group multiply bonded to the same or different nitrogen atoms in the guanidine cation; for example, an ethylene, 1,3-propylene or 1,4-butylene group.

9. A salt according to claim 8 wherein the guanidine moiety is in part of a heterocyclic structure.

10. A salt according to claim 9 wherein the structure includes one, two, or three heterocyclic rings.

11. A salt according to any one of the preceding claims wherein the anion is selected from the group consisting of *bis*-trifluoromethanesulfonyl imide, trifluoromethanesulfonyltrifluoroacetyl imide and trifluoromethanesulfonylpentafluoroethanesulfonyl imide PF₆⁻, BF₄⁻ , (CF₃SO₃⁻), AsF₆⁻, SbF₆⁻, ClO₄⁻, O₂⁻, AlCl₄⁻, GaCl₄⁻, C(SO₂CF₃)₃⁻, N(SO₂CF₃)₂⁻, SCN⁻, O₃SCF₃⁻, C₆F₅SO₃⁻, O₂CCF₃⁻, SO₆F⁻, B(C₆H₅)4⁻ *clo*s*o*carborates, *closo*borates, halogenated *closo*carborates, halogenated *closo*borates, and mixtures thereof.

12. A salt according to any one of the preceding claims which exhibits a liquid phase at 60°C, or less.

13. A salt according to claim 1 wherein the cation is derived from tetramethylguanidine hydrochloride and the anion is derived from lithium *bis*-trifluoromethanesulfonyl imide.

14. An electrolyte which includes a salt according to any one of the preceding claims.

15. An electrolyte according to claim 14 wherein the electrolyte is a gel electrolyte and includes an unsaturated monomer selected from the group consisting of dipentaerythritol hexaacrylate (DPHA), dipentaerythritol pentaacrylate (DPAA), pentaerythritol tetraacrylate, ethoxylated pentaerythritol tetraacrylate, di(trimethylolpropane) tetraacrylate (DTMPTA), trimethylolpropane trimethacrylate, ethoxylated trimethylolpropane triacrylate (ETMPTA), ethoxylated bisphenol diacrylate, hexanediol diacrylate, and mixtures thereof.

16. An electrochemical cell, which comprises:
(a) a negative electrode of either lithium or having a material capable of intercalating and de-intercalating lithium;
(b) a positive electrode comprising a cathode active material capable of intercalating lithium or capable of intercalating and de-intercalating lithium;
(c) a separator disposed between the negative and positive electrodes to prevent direct physical contact between them;
(d) an electrolyte according to claim 14 or claim 15; and
(e) a casing housing the negative and positive electrodes activated by the electrolyte.

17. Use of a salt according to any one of claims 1 to 13 or an electrolyte according to claim 14 or 15 for activating an electrochemical power source selected from the group consisting of a primary electrochemical cell, a secondary electrochemical cell, and a capacitor, the electrolyte comprising:
(a) a guanidine moiety;
(b) at least one organic substituent on the moiety; and
(c) an anion.

18. Use according to claim 17 wherein the capacitor is of either an electrolytic or a electrolytic/electrochemical hybrid type.
